# EUROPEAN PATENT APPLICATION

(11) **EP 2 263 530 A1**
(43) Date of publication of application: **22.12.2010**
(21) Application number: 09162759.6
(22) Date of filing: 16.06.2009
(51) Int. Cl.: A61B 5/08, A61B 5/11, A61M 21/00

(54) **Intelligent power-saving device**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention provides a method for controlling the power consumption of an arrangement of devices (2) in dependency to the sleep status of a person, wherein the method comprises the steps of: detecting if the person has fallen asleep, and lowering the power consumption of the arrangement of devices via at least one intermediate step. Furthermore, an apparatus (7) to carry out the method of the invention and a system (1) comprising the apparatus of the invention are disclosed.

## Description

### FIELD OF THE INVENTION

The invention relates to the field of intelligent power-saving devices wherein the power consumption of such devices is reduced and/or switched off in a gentle way without disturbing or waking-up a person fallen asleep in front of such a device. Furthermore, methods and apparatuses for the controlling of the power consumption of such devices are disclosed.

### BACKGROUND OF THE INVENTION

It is well known that some people are very susceptible to falling asleep while watching television and/or listening to the radio, i.e. while using consumer electronics. Furthermore, many people even need such consumer electronics as a means of falling asleep.

Often, people that have fallen asleep in front of, e.g., the television wake up in the morning with the television still switched on. This may mean hours of unnecessary power consumption of such devices which is neither economically nor environmentally reasonable.

Many parents use consumer electronic devices, like audio books or music, to make children fall asleep easier. It is common that older children listen to music or watch television when going to sleep. Parents often want to switch off these devices in children's room before going to sleep themselves.

In order to overcome this drawback, it is possible to construct systems that detect whether a person is awake or whether a person has fallen asleep and subsequently switch off the television.

However, major problems exist which are brought along by such a system. The main problem resides in the fact that a person that has fallen asleep is woken up by the device that is switched off. The reason for this effect is the fact that the human sensory system is sensitive to rapid changes in the environment. It is also acommon experience of parents that switching off an audio equipment in young child's room too quickly may make the child wake up. In addition, the switching-off of the device often leads to transient noises, i.e. transient effects in the audio signal generated by the device or changes in an light or heat condition which may also cause the person to wake up.

### SUMMARY OF THE INVENTION

It is an object of the invention to provide a method for controlling the power consumption of an arrangement of devices in dependency to the sleep status of a person. It is a further object of the present invention to provide an apparatus to carry out such a method.

The object of the present invention is achieved by a method according to claim 1. In particular, a method for controlling the power consumption of an arrangement of devices in dependency to the sleep status of a person is disclosed, the method comprising the steps of: a) detecting if the person has fallen asleep, and b) lowering the power consumption of the arrangement of devices via at least one intermediate step. The object of the present invention is also achieved by the apparatus according to claim 10.

The present invention is based on the key idea that the power consumption of the arrangement of devices is not immediately switched off but is rather lowered via at least one intermediate step so that the human sensory system of a person that has fallen asleep is not affected by rapid changes. Consequently, the quality of sleep and/or of the power nap of the person is improved while energy consumption is lowered, i.e. energy is saved. In a preferred embodiment the devices may be switched on again when the person wakes up, for example, if the person has programmed the device in such way. If the device determines that a person is asleep and starts the power saving procedure and if it turns out that the person was actually awake the changes in the controls of the devices may return to the original settings, preferably in a gradual manner.

In a first aspect the present invention is directed to a method for controlling the power consumption of an arrangement of devices in dependency to the sleep status of a person.

The term "controlling" is meant to refer to the ability of regulating the power consumption of the arrangement of devices. As will be detailed below, such a controlling may entail the controlling of the power consumption of one or more than one of the devices comprised by the arrangement of devices, preferably by controlling the volume of the audio signal and/or the brightness of the video signal.

The term "power consumption" refers to the amount of power, preferably electrical power that is actually consumed by the arrangement of devices, i.e. the actual current drain of the arrangement of devices.

The term "in dependency to the sleep status of a person" refers to the ability to detect whether a person is awake or has fallen asleep and to control the power consumption of the arrangement of devices based on these at least two possible states. In a preferred embodiment the different sleeping stages of the person, which are known to the skilled person, are detected and the controlling is based on the detected sleep stage. For example, if the N1 sleep stage is detected that refers to the transition of the brain from alpha waves having a frequency of 8 to 13 Hz - common in the awake state - to theta waves having a frequency of 4 to 7 Hz the control of the power consumption may be advantageously affected. In another example the rapid eye movement (REM) sleep state may be detected and the control of the power consumption may be carried out. It is further preferred that the lowering of the power consumption is based on the sleep status of the person.

In a preferred embodiment one of these two states has to be detected for a predetermined period of time before the power consumption of the arrangement of devices is controlled. More preferably, the person must have fallen asleep for ≥ 1 min and ≤ 60 min, ≥ 3 min and ≤ 45 min, ≥ 5 min and ≤ 30 min, ≥ 10 min and ≤ 40 min, or ≥ 15 min and ≤ 30 min before the control of the power consumption of the arrangement of devices is carried out.

The arrangement of devices may comprise any device that consumes power, preferably electrical power. Even more preferably the device sends out an audio signal, e.g. using an electro-acoustic transducer, i.e. a loudspeaker, and/or a video signal and/or generates heat.

In a preferred embodiment the arrangement of devices comprises at least one device, more preferably, the arrangement of devices comprises more than one device, for example ≥ 1 devices, ≥ 2 devices, ≥ 3 devices, ≥ 5 devices, or ≥ 10 devices. The devices comprised by the arrangement of devices may preferably be connectible and/or connected to each other. However, some or all of the devices may also not be connectible and/or connected to each other.

In another embodiment of the invention the arrangement of devices comprises at least one device that sends out a light, an audio signal, a video signal and/or is generating heat.

The audio signal may be any audio signal known to the skilled person. For simplicity, the term "audio signal" according to the present invention comprises the analog or digital signal that is, e.g., transferred between components of the arrangement of devices as well as the audible audio signal that is generated by a device. Preferably, such a device to play back the audio signal is an amplifier and/or an electro-acoustic transducer such as a loudspeaker that produces sound detectable by the human ear, more preferably the frequencies of the sound are in the range of ≥ 1 Hz and ≤ 30000 Hz, even more preferably in the range of ≥ 16 Hz and ≤ 20000 Hz. In further preferred embodiments the audio signal comprises music, speech, sound, a radio program, and/or noises.

The video signal may be any video signal known to the skilled person. For convenience, the term "video signal" according to the present invention comprises the analog or digital signal that is, e.g., transferred between components of the arrangement of devices as well as the visible video signal that is generated by a device converting the digital or analog signal. Preferably, the video signal is rendered visible in the user's environment using a display device producing light and/or a device which changes the propagation or reflection of light in such a way that the image represented by the video signal becomes visible to the user. The wavelength of visible light is in the range of ≥ 250 nm and ≤ 800 nm, even more preferably in the range of ≥ 380 nm and ≤ 780 nm. In further preferred embodiments the video signal comprises pictures, movies, colors, a television program and/or visual noise. The video signal can also be used to control any kind of light emitted by the device, preferably a colored light.

The device generating heat preferably is a heating or air-conditioning device. As such conventional devices are noisy lowering the heating or cooling level will advantageously decrease the noise as well. When the person is sleeping, lowering the temperature, fan speed and/or the heating level of the heating device may help to lower the power consumption. Likewise, lowering cooling level and/or fan speed of the air-conditioning device may help to lower the power consumption.

In preferred embodiments the device sending out the audio signal is a radio, i.e. a tuner, a computer, a monitor, a player device, such as an mp3 player, a cell phone, a television. Preferably, the audible sound is produced by an electro-acoustic transducer which typically comprises at least an amplifier circuit and a loudspeaker. Sound can also be produced by a heating or air-conditioning device.

In other preferred embodiments the device sending out the video signal is a computer, a player device, such as an mpeg4 player, a cell phone, a monitor. Preferably, the video signal is rendered visible using a display device, such as a television, a monitor, or a lighting device, such as a lamp.

In especially preferred embodiments the device producing the sound, the visible effect and/or generating heat is a television set equipped with the "ambilight" feature as commercially available from Philips.

The detection whether the person has fallen asleep can be accomplished by any means known to the skilled person. In specific embodiments the detecting comprises a procedure selected from the group consisting of detecting if the physiological state of the person has changed, detecting if at least one eye, preferably both eyes, of the person is shut, detecting whether REM occurs, detecting if the person snores, detecting if the breathing rate of the person changes, detecting if the heart rate of the person changes and/or detecting if the person is motionless.

The detection can be carried out using any kind of suitable sensor known to the skilled person. In specific embodiments the detection is carried out using a video capturing device, such as a video camera, an audio capturing device, such as a microphone, and/or smart textiles or smart furniture, such as cloth and/or furniture comprising, e.g., pressure sensors or motion detection sensors. For example, the video capturing device can be utilized to detect whether the eyes of the person are shut or open, the audio capturing device can be utilized to detect whether the person snores or does not snore or breathes more slowly or faster and/or the smart textiles can be utilized to detect whether the person breathes more slowly or faster and/or moves more or less frequent.

The sensor may be adapted to process the obtained data by itself. However, the detection whether the person has fallen asleep may also comprise obtaining readings from a sensor as detailed above and processing such data using a processing means, such as a microcontroller.

In a further preferred embodiment of the invention the lowering of the power consumption is only carried out after the person has fallen asleep for a predetermined amount of time. This has the advantage that the lowering of the power consumption is only carried out if the person has reliably fallen asleep. The power consumption of the arrangement of devices will not be lowered at a time when the person has not really fallen asleep. As described above, the sleeping status of the person has to be detected for a predetermined period of time before the power consumption of the arrangement of devices is controlled. For example, the person must have preferably fallen asleep for ≥ 1 min and ≤ 60 min, ≥ 3 min and ≤ 45 min, ≥ 5 min and ≤ 30 min, ≥ 10 min and ≤ 40 min, or ≥ 15 min and ≤ 30 min before the control of the power consumption of the arrangement of devices is carried out, wherein the time may vary from person to person.

The lowering of the power consumption of the arrangement of devices may be carried out by any suitable means known to the skilled person. This has the advantage that the overall power consumption of the arrangement of devices is decreased and energy is saved.

The power consumption of the arrangement of devices can be lowered by a simultaneous and/or sequential lowering of the power consumption of the devices comprised by the arrangement of devices.

A sequential lowering of the power consumption means that the power consumption of one device comprised by the arrangement of devices is lowered, preferably to a stand-by or off-state, followed by the lowering of the power consumption of a second device comprised by the arrangement of devices, preferably to a stand-by or off-state. A "stand-by" state comprises any predetermined state, wherein less energy is consumed by a device than is during regular operation. This has the advantage that the changes detectable by the human sensory system are kept minimal.

A simultaneous lowering of the power consumption means that the power consumption of at least two devices and preferably all devices comprised by the arrangement of devices is lowered at the same time, preferably to a stand-by or off-state. This has the advantage that energy savings will be maximized. It will be apparent to the skilled person that a combination of a sequential and a simultaneous lowering of the power consumption can be carried out.

In another specific embodiment of the invention the lowering of the power consumption comprises lowering and/or shutting down the audio signal and/or lowering and/or shutting down the video signal of at least one device of the arrangement of devices. Preferably, "lowering of the audio signal" means decreasing the volume of the audible audio signal, i.e. a lowering of the sound playback level and/or decreasing the level of the audio signal, i.e. decreasing the numeric values of the audio signal. Furthermore, "lowering of the video signal" preferably means a decrease in the brightness and/or luminescence of the visible video signal produced by the display device used to visualize the video signal and/or decreasing the level of the video signal, i.e. decreasing the numeric values of the video signal. Thus, preferably both the audio and the video signals may itself be altered by decrease of the numeric values of the signal and/or the gain of the device, e.g. an amplifier, converting the signals into audible and visible signals, respectively, may be reduced so that a lower playback level, brightness and/or luminescence, respectively is achieved.

In preferred embodiments the audio and/or video signal is lowered by ≥ 1 % and ≤ 100 %, ≥ 5 % and ≤ 90 %, ≥ 10 % and ≤ 80 %, ≥ 20 % and ≤ 70 %, ≥ 30 % and ≤ 60 %, or ≥ 40 % and ≤ 50 %.

In further specific embodiments of the invention the lowering of the power consumption is carried out until at least one device of the arrangement of the devices is completely switched off, set into a standby-mode and/or does no longer send out an audio and/or a video signal. Is further preferred that the at least one device is switched on again if the person wakes up again, e.g. controlled by a program. It is furthermore possible that the power consumption is restored to its original values and/or increased gradually in case the person has not fallen asleep at all, i.e. was actually awake and e.g. temporarily motionless.

In a preferred embodiment of the invention the video signal of at least one, preferably all devices comprised by the arrangement of devices is lowered first, preferably followed by a step wherein at least one, preferably all devices are switched-off or set into standby-mode. This has the additional advantage that the sleeping person is not disturbed by intense changes in the lighting during the switching-off of the devices.

In an especially preferred embodiment of the invention the audio signal of at least one, preferably all devices comprised by the arrangement of devices is lowered first, preferably followed by a step wherein at least one, preferably all devices are switched-off or set into standby-mode. This has the additional advantage that the sleeping person is not disturbed by transients of the audio system and/or by other abrupt changes in the environment that may arise during the switching-off of the devices.

The present invention is based on the unexpected finding that a lowering of the power consumption of the arrangement of devices via at least one intermediate step leads to an enhancement of the sleeping quality of the person while at the same time allowing for the saving of energy. The term "via at least one intermediate step" as used by the present invention means that the power consumption of the arrangement of devices is not digitally switched to zero, i.e. that the power consumption of the arrangement of devices is lowered stepwise. The lowering of the power consumption can be triggered by the sleep status of the person. Considering that in certain sleep stages people are less sensitive to changes in the environmental conditions, lowering of the devices can be performed during these stages. The changes in signal characteristics during these stages may be bigger. For the sleep stages, where the person is more alert and more sensitive, the changes in the environmental conditions should be minimal.

The intermediate step may be characterized by any suitable power consumption of the arrangement of devices that leads to an enhancement of the sleeping quality of the sleeping person. Generally, the power consumption of the arrangement of devices during the intermediate step is lower than the power consumption of the arrangement of devices before the lowering of the power consumption has been started and is larger than the power consumption of the arrangement of devices after the intermediate step has been passed. For example, if the power consumption of the arrangement of devices is 100 % before the person has fallen asleep and is 0 % after the lowering of the power consumption has been carried out, then the power consumption of the arrangement of devices during the intermediate step may be in the range of < 100 % and > 0 %.

In preferred embodiments the intermediate step is characterized by a power consumption that is ≥ 1 % and < 100 %, ≥ 5 % and ≤ 90 %, ≥ 10 % and ≤ 80 %, ≥ 20 % and ≤ 70 %, ≥ 30 % and ≤ 60 %, or ≥ 40 % and ≤ 50 % of the power consumption of the arrangement of devices before the lowering of the power consumption.

In a preferred embodiment, the power consumption is lowered by more than one intermediate step, preferably by ≥ 1, ≥ 2, ≥ 3, ≥ 4, ≥ 5, or ≥ 10 intermediate steps. This has the advantage that the power consumption can be lowered more subtle, thus leading to more subtle changes detectable by the human sensory system.

Generally, the power consumption can be lowered in any non-parametric manner. In even more preferred embodiments the power consumption is lowered gradually, exponentially and/or linearly. By this, a continuous lowering of the power consumption is meant, preferably wherein the change between two subsequent power consumption levels is ≤ 50 %, more preferably ≤ 15 %, and/or ≤ 5 % and most preferably no longer distinguishable by a human being.

In another embodiment the method according to the invention further comprises the step of identifying if a person has entered an area nearby the arrangement of device prior to starting the detection. This has the advantage that the method according to the invention is only carried out if a person is actually present and/or close to the arrangement of devices. Advantageously, more energy can be saved as the sensors used to, e.g., monitor the sleeping state of the person can be kept switched-off as long as no person is in the vicinity of the arrangement of devices.

The identification if a person has entered an area nearby the device may be carried out by any suitable means known to the skilled person and preferably comprises obtaining readings from a sensor, obtaining readings from a video capturing device and/or obtaining readings from an audio capturing device.

In an additional embodiment the method according to the invention further comprises the step of c) signalizing that the person has fallen asleep and/or that the power consumption of the arrangement of devices has been lowered. By "signalizing" is meant that the sleeping state of the person is communicated, preferably to a further person. This is especially advantageous in a medical environment and/or for the surveillance of children, elderly and/or chronically ill. This way such persons may be positioned in front of the arrangement of devices, e.g. a television, when the care personnel and/or the parents need to attend to different matters. This has the benefit that the subjects in front of the television are usually relatively calm and care-free for an extended period of time. The detection that the subject has fallen asleep and/or that the power consumption of the arrangement of devices has been altered may be used as a non-intrusive signal for the care personnel and/or the parents that the subjects need attention.

In a further aspect the present invention is directed to an apparatus for carrying out the method according to the present invention. Particularly the present invention discloses an apparatus for controlling the power consumption of an arrangement of devices in dependency to the sleep status of a person, wherein the apparatus comprises a detection unit for detecting if the person has fallen asleep and wherein the apparatus is adapted for lowering the power consumption of the arrangement of devices if the detection unit has detected that the person has fallen asleep.

The skilled person will readily realize the features of the apparatus according to the present invention when studying the disclosure of the method according to the invention above. However, in the following some preferred embodiments of the apparatus will be detailed.

The detection unit of the apparatus may comprise any suitable means to detect the sleeping state of the person. In one embodiment of the invention the detection unit of the apparatus comprises a suitable sensor known to the skilled person. In specific embodiments the detection unit comprises a video capturing device, such as a video camera, an audio capturing device, such as a microphone, and/or smart textiles or furniture comprising, e.g., pressure sensors or motion detection sensors. For example, the video capturing device can be utilized to detect whether the eyes of the person are shut or open, the audio capturing device can be utilized to detect whether the person snores or does not snore or breathes more slowly or faster and/or the smart textiles can be utilized to detect whether the person breathes more slowly or faster and/or moves more or less frequent.

The sensor comprised by the detection unit may be adapted to process the obtained data by itself, wherein the detection unit may be operated in an energy efficient way. However, the detection unit may also obtain the readings from a sensor as detailed above and process such data using a processing means, such as a microcontroller. In such embodiments the sensors, such as the capturing devices may be placed outside the apparatus and are preferably in connection with the apparatus. Most preferable the connection is a wireless connection.

The apparatus is adapted to carry out the lowering of the power consumption of the arrangement of devices by any suitable means. In preferred embodiments the apparatus is connectible and/or connected to the arrangement of devices and/or at least one device comprised by the arrangement of devices.

In a specific embodiment the connection between the apparatus and the arrangement of devices is achieved by a wired, preferably a wireless connection, even more preferably by use of remote control codes, such as RC5 and/or RC6 known to the skilled person. In such cases the apparatus and the arrangement of devices needs to comprise at least one transmitter and at least one receiver for the wireless connection, respectively. Advantageously, most consumer electronics are already equipped with such a receiver, e.g. an IR-receiver or a bluetooth receiver.

In a preferred embodiment of the invention the apparatus is adapted to simultaneous and/or sequential lower the power consumption of the arrangement of devices.

More preferably the apparatus is adapted to lower the power consumption of the arrangement of devices by lowering and/or shutting down the audio signal and/or lowering and/or shutting down the video signal of at least one device of the arrangement of devices.

In another preferred embodiment the apparatus is adapted to completely switch off at least one of the devices comprised by the arrangement of devices and/or set at least one of said devices into a stand-by state.

In another preferred embodiment of the invention the apparatus is adapted to lower the power consumption of the arrangement of devices gradually, exponentially and/or linearly.

In another specific embodiment of the invention the apparatus further comprises an identification unit for identifying if the person has entered an area nearby the arrangement of devices and wherein the identification unit is preferably adapted for notifying the detection unit if the person has entered the area nearby the device. As explained above, such a configuration may advantageously lead to an increase in power savings.

In a further embodiment the apparatus further comprises a signaling means to signal that the subject has fallen asleep and/or that the power consumption of the arrangement of devices has been lowered. While this signaling may be carried out by any suitable means it is preferably carried out wirelessly.

In another aspect the present invention is directed to a system comprising an arrangement of devices as detailed above and an apparatus according to the invention, wherein the apparatus is adapted for lowering the power consumption of the arrangement of devices in dependency to the sleep status of a person. The skilled person will readily realize the features of the system according to the present invention when studying the disclosure of the method and apparatus according to the present invention above.

In another aspect the present invention is directed to the use of the apparatus and/or the system of the present invention in a medical environment.

### BRIEF DESCRIPTION OF THE DRAWINGS

These and other aspects of the invention will be apparent from and elucidated with reference to the embodiments described hereinafter.

In the drawings:

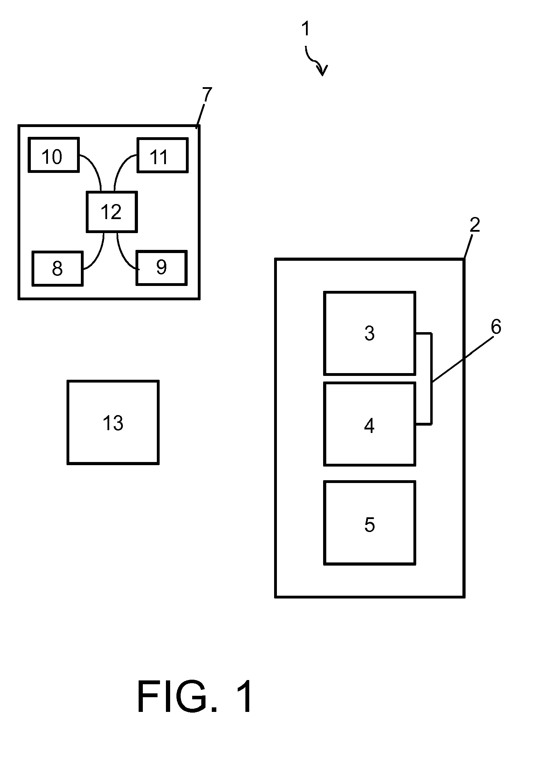

- Fig. 1: shows a schematic view of a system according to the invention.

### DETAILED DESCRIPTION OF EMBODIMENTS

Fig. 1 is a schematic view of a system 1 according to the invention. The system 1 comprises an arrangement of devices 2, an apparatus according to the invention 7 and a piece of smart furniture 13, equipped with a pressure sensor.

The arrangement of devices 2 comprises a television set 3 that sends out and audio signal through its speakers and a video signal through its display. Furthermore, the television is equipped with the "ambilight" feature known from Philips, i.e. the television set additionally sends out light of a color that matches the contents displayed the display.

The arrangement of devices 2 further comprises a radio 4, i.e. a tuner that sends out an audio signal. The television 3 and the tuner 4 are connected by a wired connection 6.

The arrangement of devices further comprises a lighting device 5, i.e. a lamp that is color tuneable and/or dimmable.

The television 3, the radio 4 and the lamp 5 all contain a wireless sensor operating in the GHz region and can be controlled by means of a single remote control (not shown).

The apparatus according to the invention 7 comprises a camera 8, a means 9 to control the power consumption of the arrangement of devices 2, a detection unit 10 and an identification unit 11. Furthermore, the apparatus comprises a microcontroller 12 connected to camera 8, means for control of power consumption 9, identification unit 11 and detection unit 10.

The camera 8 is a sensor, i.e. a regular video capturing device used to monitor the person sitting in front of the arrangement of devices 2.

The means 9 to control the power consumption of the arrangement of devices is embodied as a remote control sending unit operating in the GHz region and capable of individually controlling the television 3, the radio 4 and the lamp 5 using the RC6 standard.

The detection unit 10 is obtaining the readings from the camera 8, the smart textiles worn by the person sitting in front of the arrangement of devices (not shown) and the piece of smart furniture 13. The connection between detection unit, smart textiles and smart furniture 13 is a wireless connection. Using the microcontroller 12 it is detecting whether the person is in a sleeping state for a predetermined time of at least 10 minutes.

The identification unit 11 is in wireless connection with the piece of smart furniture 13, which is embodied as a couch comprising pressure sensors that are activated as soon as a person sits down on the couch. In a state wherein no person is sitting on the couch for more than 10 minutes the identification unit 11 powers off the remaining components of the apparatus 7 in order to save energy. If afterwards a person sits down on the couch 13, the apparatus 7 is brought back into the fully powered operational state.

In an exemplary scenario, a person sits down on the couch 13 in front of the arrangement of devices 2 and switches on the television set 3 and the lamp 5.

Due to the detection of pressure, the pressure sensors comprised by the couch 13 transmit a wireless signal to the identification unit 11 of the apparatus 7 that is consequently brought into an active state and starts monitoring the eyes and movements of the person sitting on the couch by means of the camera 8. Additionally, the person is wearing smart textiles comprising motion sensors which are wirelessly connecting to the identification unit 10 of the apparatus. Likewise, the pressure sensors of the couch 13 establish a wireless connection with the detection unit 10 of the apparatus 7.

After the apparatus 7 detects that the person sitting in front of the arrangement of devices has fallen asleep, i.e. by detection of shut eyes and less frequent movements of the person for a period of at least 10 minutes starts controlling the lowering of the power consumption of the arrangement of devices 2 by means of the sending unit 9 controlled by the microcontroller 12.

In a first step, the audio signal of the television 3 is gradually lowered until the volume is completely turned off. In a next step the video signal of both the television 3 and the lamp 5 are gradually lowered simultaneously to slowly reduce the light in the room the person is in.

The television 3 is then brought into a stand-by mode, while the color of the lamp 5 is tuned to a warm color. The lamp is not completely turned off but dimmed to a very low brightness so that when the person awakes an orientation in the room is possible.

In a last step, the sending unit 9 is then instructed by the microcontroller 12 to signalize to a further person (not shown) that the person sitting in front of the arrangement of devices has fallen asleep and that the power consumption of the arrangement of devices has been lowered.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. Method for controlling the power consumption of an arrangement of devices (2) in dependency to the sleep status of a person, the method comprising the steps of:
a) detecting if the person has fallen asleep, and
b) lowering the power consumption of the arrangement of devices via at least one intermediate step.

2. The method according to claim 1, wherein the arrangement of devices comprises at least one device sending out a light, an audio signal and/or a video signal and/or generating heat.

3. The method according to claim 1, wherein detecting if the person has fallen asleep comprises a procedure selected from the group consisting of detecting if the physiological state of the person has changed, detecting if at least one eye of the person is shut, detecting whether REM occurs, detecting if the person snores, detecting if the breathing rate of the person changes, detecting if the heart rate of the person changes and/or detecting if the person moves less frequently.

4. The method according to claim 1, wherein the lowering of the power consumption is carried out after the person has fallen asleep for a predetermined amount of time.

5. The method according to claim 1, wherein the lowering of the power consumption of the arrangement of devices is carried out sequentially and/or simultaneously.

6. The method according to claim 1, wherein the lowering of the power consumption comprises lowering and/or shutting down the audio signal and/or lowering and/or shutting down the video signal and/or lowering the heating and/or lowering the cooling level and/or lowering the light of at least one device of the arrangement of devices.

7. The method according to claim 1, wherein the lowering of the power consumption comprises first lowering the audio signal of at least one device of the arrangement of devices followed by a switching-off of said device.

8. The method according to claim 1, wherein the lowering of the power consumption is carried out gradually, exponentially, linearly and/or according to the sleep status of the person.

9. The method according to claim 1, further comprising the step of identifying if the person has entered an area nearby the arrangement of devices prior to starting the detection.

10. The method according to claim 1, further comprising the step of:
c) signalizing that the person has fallen asleep and/or that the power consumption of the arrangement of devices has been lowered.

11. Apparatus (7) for controlling the power consumption of an arrangement of devices (2) in dependency to the sleep status of a person, comprising
a detection unit (10) for detecting if the person has fallen asleep; and wherein
the apparatus is adapted for lowering the power consumption of the arrangement of devices if the detection unit has detected that the person has fallen asleep.

12. The apparatus according to claim 10, wherein the apparatus is adapted to lower the power consumption of the arrangement of devices gradually, exponentially and/or linearly.

13. The apparatus according to claim 10, further comprising an identification unit (11) for identifying if the person has entered an area nearby the device and wherein the identification unit is preferably adapted for notifying the detection unit if the person has entered the area nearby the device.

14. A system (1) comprising an arrangement of devices (2) and an apparatus (7) according to claim 10 or 11, wherein the apparatus is adapted for lowering the power consumption of the arrangement of devices in dependency to the sleep status of a person.
